# EUROPEAN PATENT APPLICATION

(11) **EP 2 228 451 A1**
(43) Date of publication of application: **15.09.2010**
(21) Application number: 09154931.1
(22) Date of filing: 11.03.2009
(51) Int. Cl.: C12Q 1/68

(54) **Method for determining the primary site of cup**

(71) Applicant: Universiteit Maastricht, 6211 LK Maastricht (NL); Academisch Ziekenhuis Maastricht, 6229 HX Maastricht (NL)
(72) Inventor: Ayoubi, Torik Adnen Yusif, 3600 Genk (BE); Stassen, Alphonsius Petrus Marie, 6219 BG Maastricht (NL)
(74) Representative: Habets, Winand

(57) **Abstract**

The invention relates to a method for the classification of cancer using a specific multi-class tumor classifier comprising specific sets of genes for the interpretation of expression data obtained from tumor samples. More specifically, the method of the present invention provides an accurate, reproducible, robust, objective and easy to perform method for determining the primary site of a Cancer of Unknown Primary site (CUP). For this purpose, the method provides that a classifier parameter is determined by comparing an expression profile of a tumor sample with a template profile representative for a particular primary site of a cancer.

## Description

### FIELD OF THE INVENTION

The invention relates to a method for the classification of cancer using a specific multi-class tumor classifier comprising specific sets of genes for the interpretation of expression data obtained from tumor samples. More specifically, the method of the present invention provides an accurate, reproducible, robust, objective and easy to perform method for determining the primary site of a Cancer of Unknown Primary site (CUP). For this purpose, the method provides that a classifier parameter is determined by comparing an expression profile of a tumor sample with a template profile representative for a particular primary site of a cancer.

### BACKGROUND OF THE INVENTION

Traditional cancer diagnosis relies on a combination of clinical and histopathological data that differ from hospital to hospital and even from pathologist to pathologist. The interpretation of histopathological studies is based on morphology of the tumor and the cell types that it consists of. These classic approaches may fail when dealing with atypical tumors or morphologically indistinguishable tumor subtypes. Immunohistochemical approaches bring in an extra level of information as besides morphological information also expression of particular markers can be incorporated in the analysis. In many cases, however, the morphology of in particular metastatic cancer is not discriminative enough. The large majority of metastatic carcinomas arise in lung, colon, breast, prostate, stomach, and pancreas. In general, these metastatic carcinomas have very similar morphological features and microscopic appearances do not provide enough discriminative power for diagnosis of their site of origin.

Metastatic Cancer of Unknown Primary site (CUP) is one of the 10 most frequent cancer diagnoses worldwide, and constitutes 3-4% of all human malignancies. Patients with CUP present themselves at the clinic with metastatic disease without an identifiable primary tumor and for which the primary tumor site remains unknown even after extensive attempts to determine the site of tumor origin. Effective treatment of cancer, however, fundamentally depends on the primary anatomical site of the tumor and, therefore, determination of cancer subtype is important for optimal cancer management and therapy.

Very recently, investigators of the MD Anderson Comprehensive Cancer Center (Houston, Texas, USA) have reported that the one-treatment-fits-all approach to CUP is no longer valid. Their findings (Varadhachary et al., 2008, Carcinoma of unknown primary with a colon-cancer profile-changing paradigm and emerging definitions. Lancet Oncol. 9:596-9) suggest that patients with CUP with a colon-cancer profile derive substantial benefit from the use of specific treatments developed for colon cancer. They conclude that in the era of molecular profiling, it is expected that additional work with other CUP subsets will provide attractive tailored treatment alternatives, with efficacies that exceed the current one-treatment-fits-all approach.

In a study to assess the process by which a pathologist can identify the origin of a metastatic carcinoma of unknown primary site, 100 metastatic adenocarcinomas were presented to two pathologists as carcinomas of unknown primary site. The correct primary site was chosen as primary choice in only 49% of the cases (Sheahan et al 1993). These results indicate that if the primary site should continue to contribute to treatment decisions, more accurate and objective methods have to be developed for the diagnosis of tumor origin of cancers of unknown primary site.

Studies have already demonstrated that it is possible to use microarray data in the prediction of the tissue of origin of tumors and specific gene subsets were identified having an expression profile typical for each cancer class.

However, several of these studies have shown that simple unsupervised clustering based on the most variable expressed genes was not able to separate all the tumors. This indicates that even tumors of different histological origins have, in general, highly similar expression patterns as to impede the use of simple hierarchical clustering techniques. Another important observation was that these tumors could not be accurately classified according to their tissue of origin and that poorly differentiated tumors do not simply lack a few key markers but have a fundamentally distinct gene expression pattern.

With the developed approaches a classification method with an accuracy of about 77% could be obtained.

Recent studies have thus provided a proof-of-concept that microarray gene expression profiling can be used to classify cancer subtypes. However, most of these studies have based their analysis on a particular set of carefully selected representative tumors and developed a classifier on that particular set of tumors.

The problem is that these studies use one particular tumor set for the development of a classifier, whereas for the development of a more general classifier other tumor sets have to be taken into consideration for the reason that gene sets derived from one particular tumor set in one study differed significantly from those from the other studies.

In view hereof, there remains a pressing need to develop an accurate, reproducible, robust and easy to perform method for the classification of CUPs. Indeed, it is for instance very difficult to search for efficient therapies against very heterogeneous tumors. In contrast, a reliable classification of CUP would allow to provide targeted therapies for each tissue related tumor. A reliable and easy to perform classification method would then allow to choose for each patient an adapted treatment depending on the site of origin of the tumor.

In particular, the prognosis for CUP is very heterogeneous. Currently, the main treatment of CUP is the surgical removal of the tumor if possible, which may be followed by adjuvant chemotherapy. Chemotherapy may be very tiresome and painful for patients but is necessary in case of CUP with poor prognosis. A classification and prognosis method of CUP would thus also be very helpful to decide whether or not to administer an adjuvant therapy to a patient.

In the present invention it has been unexpectedly found that a specific multi-class tumor classifier comprising specific sets of genes can be used to interpret microarray data obtained from tumor samples thereby determining the primary site of the CUP with an accuracy larger than 80%.

### SUMMARY OF THE INVENTION

The present invention bridges the gap between traditional classification methods for tumors, and classification methods based on molecular biology assays. The method of the present invention thereby provides an accurate, reproducible, robust, objective and easy to perform method for determining the primary site of a Cancer of Unknown Primary site (CUP). For this purpose, the method provides that a classifier parameter is determined by comparing an expression profile of a tumor sample with a template profile representative for a particular primary site of a cancer. Consequently, the method enables to determine the primary site of CUPs. Advantageously the method of the present invention is very consistently across a wide variety of data sets, the method is accurate and reproducible and further provides a robust and easy to perform method for the classification. The present invention further allows the identification of the primary site of poorly differentiated CUP tumors which have been known in the prior art to provide fundamentally distinct gene expression patterns, consequently making it very difficult to accurately determine the primary sites.

Accordingly, the present invention relates to a method for classifying a tumor according to the site of origin of said tumor, comprising:
(a) determining the expression profile of a sample;
(b) calculating a classifier parameter between said expression profile and a tissue-specific template; said expression profile comprising the expression levels of a plurality of tissue-specific genes in said sample; said plurality of tissue-specific genes consisting of at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54 or 55 of the tissue-specific genes for which markers are listed in Table 1 said tissue-specific template comprising, for each tissue-specific gene in said plurality of tissue-specific genes, the representative expression level of said tissue-specific gene in said tissue;
(c) classifying said tumor according to the site of origin if said classifier parameter is above a chosen threshold or if said expression profile is more similar to a tissue-specific template than to another tissue-specific template.

The present invention also relates to a microarray comprising a plurality of probes complementary and hybridisable to sequences in at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54 or 55 different genes for which markers are listed in Table 1, wherein said plurality of probes is at least 25%, 50%, 60%, 70%, 75%, 80%, 90%, 95% or 100% of probes on said microarray.

The present invention also relates to a computer system comprising a processor, and a memory coupled to said processor and encoding one or more programs, wherein said one or more programs instruct the processor to carry out the method of the present invention.

The present invention also relates to a kit for determining the site of origin of a tumor, comprising at least one microarray comprising probes to at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54 or 55different tissue-specific genes for which markers are listed in Table 1, and a computer readable medium having recorded thereon one or more programs for carrying out the method of the present invention.

These and further aspects and embodiments are described in the following sections and in the claims.

### BRIEF DESCRIPTION OF TABLES

**Table 1** provides a list of 55 tissue-specific probes. The sequence, name and tissue for which the probe is representative are provided in the table.

**Table 2** provides a list of tissues and their respective tumor subclasses.

**Table 3** provides the results of the classification of 33 tissue samples.

**Table 4** provides an overview of four samples wherein two or three tissues are mixed in a specific proportion.

**Table 5** provides the classification results of the 4 mixtures represented in Table 3.

**Table 6** provides the results of individual gene levels and demonstrates that individual gene levels can be used for classification.

**Table 7** provides correlation values between technical replicates.

### DETAILED DESCRIPTION OF THE INVENTION

Before the present method and devices used in the invention are described, it is to be understood that this invention is not limited to particular methods, components, or devices described, as such methods, components, and devices may, of course, vary. It is also to be understood that the terminology used herein is not intended to be limiting, since the scope of the present invention will be limited only by the appended claims.

Unless defined otherwise, all technical and scientific terms used herein have the same meaning as commonly understood by one of ordinary skill in the art to which this invention belongs. Although any methods and materials similar or equivalent to those described herein may be used in the practice or testing of the present invention, the preferred methods and materials are now described.

In this specification and the appended claims, the singular forms "a", "an", and "the" include plural references unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps.

The terms "comprising", "comprises" and "comprised of" also include the term "consisting of'.

The term "about" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

The recitation of numerical ranges by endpoints includes all numbers and fractions subsumed within the respective ranges, as well as the recited endpoints.

All documents cited in the present specification are hereby incorporated by reference in their entirety.

The present invention bridges the gap between traditional classification methods for tumors, and classification methods based on molecular biology assays. The method of the present invention thereby provides an accurate, reproducible, robust, objective and easy to perform method for determining the primary site of a Cancer of Unknown Primary site (CUP). For this purpose, the method provides that a classifier parameter is determined by comparing an expression profile of a tumor sample with a template profile representative for a particular primary site of a cancer. Consequently, the method enables to determine the primary site of CUPs. Advantageously the method of the present invention is very consistently across a wide variety of data sets, the method is accurate and reproducible and further provides a robust and easy to perform method for the classification.

Additionally to determining the primary site of a CUP, the present invention provides information regarding the most appropriate chemotherapy treatment which would be best suited to treat the disease. This assessment of the most appropriate treatment is based on the correct determination of the correct type of tumor and/or tumor subtype involved in the disease.

The site of origin of CUP refers to the primary site of a Metastatic Cancer of Unknown Primary site (CUP). Patients with CUP present themselves at the clinic with metastatic disease without an identifiable primary tumor and for which the primary tumor site remains unknown even after extensive attempts to determine the site of tumor origin. Effective treatment of cancer, however, fundamentally depends on the primary anatomical site of the tumor and, therefore, determination of the site of origin or primary site of the cancer is important for optimal cancer management and therapy.

The present invention therefore provides methods for the classification of tumors. Such classification has many beneficial applications. For example, by associating a CUP with a specific tissue this classification may correlate with prognosis and/or susceptibility to a particular therapeutic regimen. As such, the classification may be used as the basis for a prognostic or predictive kit and may also be used as the basis for identifying previously unappreciated therapies. Therapies that are effective against only a particular tumor from one type of tissue may have been lost in studies whose data were not stratified by tissue type; the present invention allows such data to be re-stratified, and allows additional studies to be performed, so that tissue-specific therapies may be identified and/or implemented.

According to the invention, a "classification" is intended to refer to the determination for any CUP tumor of the primary site of said tumor. The present invention provides sets of tissue-specific genes and markers which have been found to be representative for a specific tissue and therefore enable the classification of a CUP tumor according to it's primary site. The term "representative" is intended to refer to distinguishing or distinctive, meaning that it serves to identify.

Identifying the primary origin of CUPs therefore provides knowledge of the survival chances of an individual having contracted cancer. It also provides insights on which sort of treatment should be offered to the individual having contracted cancer, thus providing an improved treatment response of the individual. Likewise, the individual may be spared treatment that is inefficient in treating the particular type of cancer and thus spare the individual severe side effects associated with treatment that may even not be suitable for the type of cancer.

It is likely that for a person skilled in the art, in at least some instances, identification of the site of origin of a CUP correlates with prognosis or responsiveness. In such circumstances, it is possible that the same set of interaction partners can act as both a classification panel and a prognosis or predictive panel.

The different aspects and embodiments of the present invention are further supported by non-limiting examples. Example 1 provides the method that can be used for establishing a set of tissue-specific genes or markers. Example 2 shows how the method of the present invention can be used to determining the origin of a specific tissue sample. Example 3 shows that the method also enables to determine multiple sites of origin of a mixed tissue sample. In Example 4 and 5 the reproducibility and robustness of the method of the present invention is assessed whereas Example 6 provides examples wherein the primary site of tumor tissue samples is determined using the method of the present invention.

Accordingly, within one embodiment of the present invention, a method is provided for classifying a tumor according to the site of origin of said tumor, comprising:
(a) determining the expression profile of a sample;
(b) calculating a classifier parameter between said expression profile and a tissue-specific template; said expression profile comprising the expression levels of a plurality of tissue-specific genes in said sample; said plurality of tissue-specific genes comprising at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55 or more of the tissue-specific genes for which markers are listed in Table 1; said tissue-specific template comprising, for each tissue-specific gene in said plurality of tissue-specific genes, the representative expression level of said tissue-specific gene in said tissue; and
(c) classifying said tumor according to the site of origin if said classifier parameter is above a chosen threshold or if said expression profile is more similar to a tissue-specific template than to another tissue-specific template.

As used herein, the term "expression profile" refers to a profile or pattern which is the result of the expression of one gene or more genes in a sample. The profile is characteristic of the state of the sample. In a tumor sample a plurality of gene expression products are present. By expression profile is meant the combination of a number of expression products and/or the amount of expression products specific for a given biological condition, such as cancer. The pattern is produced by determining the expression products of selected genes that together reveals a pattern or profile that is indicative of the biological condition. As used herein, the "expression profile" refers to either the expression profile at the nucleic acid level and/or the expression profile at the protein level. It should be noted that the expression of one gene or more genes in a sample can be determined either by measuring the gene expression directly. Since expressed genes are translated into proteins, the gene expression can also be measured at the protein level, such as for instance, by measuring and detecting proteins in tumor biopsies, plasma, serum or on any type of tissue as mentioned in the present application.

In the present invention, nucleic acids or the selected genes are extracted from a sample taken from an individual having cancer. The sample may be collected in any clinically acceptable manner, but must be collected such that marker-derived polynucleotides (e.g., RNA) are preserved. mRNA or nucleic acids derived therefrom (e.g., cDNA or amplified DNA) are preferably labeled distinguishably from standard or control polynucleotide molecules, and both are simultaneously or independently hybridized to a microarray comprising some or all of the markers or marker sets or subsets described above. Alternatively, mRNA or nucleic acids derived therefrom may be labeled with the same label as the standard or control polynucleotide molecules, wherein the intensity of hybridization of each at a particular probe is compared.

A sample may comprise any clinically relevant tissue sample, such as a tumor biopsy or fine needle aspirate, or a sample of bodily fluid, such as blood, plasma, serum, lymph, ascitic fluid, cystic fluid, saliva, cerebrospinal fluid, urine or nipple exudate. The sample may be taken from a human, or, in a veterinary context, from non-human animals such as ruminants, horses, swine or sheep, or from domestic companion animals such as felines and canines.

In a specific embodiment of the present invention said sample is processed, prior to performing the method of the present invention, according to any techniques known in the art. As non-limiting examples, a tumor biopsy can be preserved using techniques known in the art, such as, but not limited to, preserving the tumor biopsy using a preservative such as RNAlater^{®}, snap-freezing the tumor biopsy in liquid nitrogen, fixing the tumor biopsy in an organic solvent such as formaldehyde and paraformaldehyde, processing the tumor biopsy for histological examination or formalin-fixing and paraffin-embedding the tumor biopsy tissue.

In a preferred embodiment the sample is a sample containing Circulating tumor cells (CTC) isolated from blood. Generally in the identification of tumors, a biopsy needs to be taken from the patient. This procedure is rather unpleasant and lays a large burden on the patient when taking a biopsy form the metastatic cancer. In order to significantly reduce this burden the analysis of the present invention can be performed on CTC isolated from blood.

Another advantage associated with the method of the present invention is that it enables the analysis of Circulating tumor cells (CTC) isolated from blood. Generally in the identification of tumors, a biopsy needs to be taken from the patient. This procedure is rather unpleasant and lays a large burden on the patient when taking a biopsy form the metastatic cancer. In order to significantly reduce this burden the analysis of the present invention can be performed on CTC isolated from blood.

Technologies for isolating CTC are advancing rapidly and CTC have a great potential for biomarker research. In contrast to CT scans, which are expensive and do not provide any molecular information, and biopsies, which are difficult to serially collect, the assessment of CTC provides a readily accessed and cheaper biomarker. Application the method of the present invention on CTC will make it possible to determine dynamic changes in the tumor population, and will help to evaluate therapeutic response. The tool can also be used for to demonstrate proof of mechanism (POM) for novel drugs as changes in gene expression in the CTC will provide evidence that the tumor is responding to the drug appropriately.

Another important advantage for the method of the present invention is that the method can be used for more than one tumor type. The method of the present invention can be used for determining the site of origin of CUP's for different tumor types including carcinoma, sarcoma, melanoma central or peripheral nervous system, and lymphoma tumor classes and more preferably carcinoma tumor classes.

Other features that will be provided by the method of the present invention is whether the carcinoma is squamous of nature or if it has adenocarcinoma features and whether the adenocarcinoma is of muscinous or serous nature.

As used herein, the term "classifier parameter" represents a discriminative value that is used for the classification. The parameter is calculated using either differences in the expression level between a sample and template, or by calculation of a correlation coefficient. Such a coefficient can be calculated using for instance a Pierson correlation coefficient. By using the Pearson correlation coefficient a dimensionless index is provided, said index reflecting the extent of a linear relationship between two data sets

As used herein, a "template profile" refers to a profile obtained through measuring the expression levels of genes or markers. More specifically, as used herein, the term "tissue-specific template" refers to a template profile wherein the set of genes or markers are representative for a specific tissue or tissue-type. The tissue-specific template can further be defined as the error-weighted log ratio average of the expression difference for the group of marker genes able to identify the site of origin of a CUP. Templates are for instance defined for different tissue samples or tissue types. Additionally, the template profile may be defined as the error-weighted log ratio average of the expression difference for the group of marker genes in different tissues. Consequently, the template profile provides information regarding several tissue-types in a single profile. This enables the fast and accurate identification of the correct tissue-type out of a series of tissue-types.

The method of the present invention enables determining the primary site of CUPs. This primary site is a specific tissue which may be a tissue from the group comprising adrenal gland, bone marrow, brain, bronchiole, bronchus, bulbourethral gland, cecum, cerebellum, cerebral meninx, Colon, Duodenum, Epididymis, Esophagus, Eyeball, Gallbladder, Glandular stomach, Harderian gland, Hematopoietic tissue, Ileum, Jejunum, Joint, Kidney, Large intestin, Larynx, Liver, Lung, Mammary gland, Nasal cavity, Nasopharynx, Oral cavity, Ovary, Oviduct, Pancreas, Paranasal sinus, Parathyroid gland, Parotid gland, Pineal gland, Pituitary gland, Pleura, Preputial gland, Prostate, Rectum, Renal pelvis, Salivary gland, Seminal vesicle, Skeletal muscle, Skeletal system, Skin/subcutaneous tissue, Small intestin, Soft tissue, Spinal cord, Spinal meninx, Sublingual gland, Testis, Thymus, Thyroid gland, Tongue, Tooth, Trachea, Ureter, Urethral gland, Urinary bladder, Uterine cervix, Uterus, Vagina and/or Zymbal gland. More preferably said tissue is chosen from the group comprising breast tissue, cerebellum tissue, heart tissue, kidney tissue, liver tissue, muscle tissue, pancreas tissue, prostate tissue, spleen tissue, testis tissue, thyroid tissue. lung tissue, ovary tissue, endometrium tissue, cervix tissue, colon/rectum tissue, stomach tissue, bladder tissue, adrenal tissue, sarcoma tissue, skin tissue, lymphoma tissue and/or Central Nervous System tissue. The most preferred tissues are chosen from the group comprising the most frequently occurring types of cancer including breast, lung, colon, prostate, ovary, liver, esophagus, uterus, bladder, kidney, brain, bone marrow and/or lymphoid cancer.

In a further preferred embodiment, subclasses within the tissue classes are determined by the method of the present invention. As a matter of example, a list of tissue classes and their respective tumor types and sub-classes is provided in Table 2.

Each tissue or tissue subclass provides a set of tissue-specific genes that are representative for said tissue. Said set of tissue-specific genes is characterized by having a significantly increased expression level in said tissue, compared to other tissues. Accordingly, as used herein, "tissue-specific genes" generally refers to genes which are representative for a specific tissue, by having a significant increased expression level compared to all other tissues.

The term "marker" as used herein refers to a gene or gene products, or an EST derived from that gene, the expression or level of which changes between certain conditions. Where the expression of the gene or gene products correlates with a certain tissue, the gene or its products are a marker for that tissue.

In one embodiment, the similarity of said expression profile to said tissue-specific template is represented by a correlation coefficient between said expression profile and said tissue-specific template, respectively, and a correlation coefficient greater than a correlation threshold, e.g., 0.5, indicates a high similarity and said correlation coefficient equal to or less than said correlation threshold indicates a low similarity. In another embodiment, the similarity of said expression profile to said tissue-specific template is represented by a distance between said cellular constituent profile and said tissue-specific template, respectively, and a distance less than a given value indicates a high similarity and said distance equal to or greater than said given value indicates a low similarity. The correlation coefficient may also indicate if a certain expression profile is more similar to a certain tissue-specific template than to another tissue-specific template.

In one embodiment, the invention provides a set of 55 primary site tissue-specific markers, e.g., markers that are significantly correlated with a specific tissue. These markers are listed in Table 1. Table 1 list the markers, their SEQ ID NO:s 1 to 55, their gene names and the tissue for which each of the markers are representative. The invention also provides subsets of at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54 or 55of the different markers present in Table 1, which are useful for determining the primary site of CUPs. The invention further provides subsets of at least 10%, 15%, 20%, 25%, 30%, 40%, 50%, 60%, 70%, 80%, or 90% of the different markers listed in Table 1. Preferably, a subset comprises all 55 different markers listed in Table 1. Accordingly the 55 markers enlisted in Table 1 enable the classification of 11 tissues types. Consequently these markers also enable the classification of CUPs according to their tissue of origin.

Within another embodiment of the present invention, a method according to the present invention is provided wherein wherein step (b) is repeated for a plurality of site of origin templates, each site of origin template being representative for a specific tissue, thereby calculating a plurality of classifier parameters.

According to yet another embodiment of the present invention, a method is provided wherein the method additionally comprises the steps of:
a) isolating nucleic acids from the sample; and
b) measuring the expression levels of the isolated nucleic acids, thereby determining the expression level of a plurality of tissue-specific genes.

According to yet another embodiment of the present invention a plurality of classifier parameters are calculated. This plurality of classifier parameters can subsequently be used for the classification of the tumor.

Methods for preparing total and poly(A)+ RNA are well known and are described generally in Sambrook et al, Molecular Cloning - A Laboratory Manual (2nd Ed.), Vols. 1-3, Cold Spring Harbor Laboratory, Cold Spring Harbor, New York (1989) and Ausubel et al., Current protocols in molecular biology, Vol. 2, Current Protocols Publishing, New York (1994).

RNA may be isolated from eukaryotic cells by procedures that involve lysis of the cells and denaturation of the proteins contained therein. Cells of interest include wild-type cells (e.g., non-cancerous), drug-exposed wild-type cells, tumor- or tumor-derived cells, modified cells, normal or tumor cell line cells, and drug-exposed modified cells.

Additional steps may be employed to remove DNA. Cell lysis may be accomplished with a nonionic detergent, followed by microcentrifugation to remove the nuclei and hence the bulk of the cellular DNA. In one embodiment, RNA is extracted from cells of the various types of interest using guanidinium thiocyanate lysis followed by CsCl centrifugation to separate the RNA from DNA (Chirgwin et al, Biochemistry 18:5294-5299 (1979)). Poly(A)+ RNA is selected by selection with oligo-dT cellulose (see Sambrook et al., Molecular Cloning - A Laboratory Manual (2nd Ed.), Vols. 1-3, Cold Spring Harbor Laboratory, Cold Spring Harbor, New York (1989)). Alternatively, separation of RNA from DNA can be accomplished by organic extraction, for example, with hot phenol or phenol/chloroform/isoamyl alcohol.

If desired, RNAse inhibitors may be added to the lysis buffer. Likewise, for certain cell types, it may be desirable to add a protein denaturation/digestion step to the protocol.

For many applications, it is desirable to preferentially enrich mRNA with respect to other cellular RNAs, such as transfer RNA (tRNA) and ribosomal RNA (rRNA). Most mRNAs contain a poly(A) tail at their 3' end. This allows them to be enriched by affinity chromatography, for example, using oligo(dT) or poly(U) coupled to a solid support, such as cellulose or Sephadex(TM) (see Ausubel et al., Current protocols in molecular biology, Vol. 2, Current Protocols Publishing, New York (1994)). Once bound, poly(A)+ mRNA is eluted from the affinity column using 2 mM EDTA/0.1 % SDS.

The sample of RNA can comprise a plurality of different mRNA molecules, each different mRNA molecule having a different nucleotide sequence. In a specific embodiment, the mRNA molecules in the RNA sample comprise at least 100 different nucleotide sequences. More preferably, the mRNA molecules of the RNA sample comprise mRNA molecules corresponding to each of the marker genes. In another specific embodiment, the RNA sample is a mammalian RNA sample.

In a specific embodiment, total RNA or mRNA from cells are used in the methods of the invention. The source of the RNA can be cells of a plant or animal, human, mammal, primate, non-human animal, dog, cat, mouse, rat, bird, yeast, eukaryote, prokaryote, etc. In specific embodiments, the method of the invention is used with a sample containing total mRNA or total RNA from 1 x 10⁶ cells or less. In another embodiment, proteins can be isolated from the foregoing sources, by methods known in the art, for use in expression analysis at the protein level.

In yet another embodiment of the present invention, the method of the present invention is provided wherein SEQ ID NOa 1 to 5 are representative for breast tissue, SEQ ID NOa 6 to 10 are representative for cerebellum tissue, SEQ ID NOa 11 to 15 are representative for heart tissue, SEQ ID NOa 16 to 20 are representative for kidney tissue, SEQ ID NO:s 21 to 25 are representative for liver tissue, SEQ ID NO:s 26 to 30 are representative for muscle tissue, SEQ ID NO:s 31 to 35 are representative for pancreas tissue, SEQ ID NO:s 36 to 40 are representative for prostate tissue, SEQ ID NO:s 41 to 45 are representative for spleen tissue, SEQ ID NO:s 46 to 50 are representative for testis tissue, and SEQ ID NO:s 51 to 55 are representative for thyroid tissue.

In another preferred embodiment said plurality of tissue-specific genes are at least 1, 2, 3, 4 or 5 breast tissue-specific genes, for which markers correspond to SEQ ID NO:s 1, 2, 3, 4 and/or 5; at least 1, 2, 3, 4 or 5 cerebellum tissue-specific genes for which markers correspond to SEQ ID NO:s 6, 7, 8, 9 and/or 10; at least 1, 2, 3, 4 or 5 heart tissue-specific genes for which markers correspond to SEQ ID NO:s 11, 12, 13, 14 and/or 15; at least 1, 2, 3, 4 or 5 kidney tissue-specific genes for which markers correspond to SEQ ID NO:s 16, 17, 18, 19 and/or 20; at least 1, 2, 3, 4 or 5 liver tissue-specific genes, for which markers correspond to SEQ ID NO:s 21, 22, 23, 24 and/or 25; at least 1, 2, 3, 4 or 5 muscle tissue-specific genes for which markers correspond to SEQ ID NO:s 26, 27, 28, 29 and/or 30; at least 1, 2, 3, 4 or 5 pancreas tissue-specific genes, for which markers correspond to SEQ ID NO:s 31, 32, 33, 34 and/or 35; at least 1, 2, 3, 4 or 5 prostate tissue-specific genes for which markers correspond to SEQ ID NO:s 36, 37, 38, 39 and/or 40; at least 1, 2, 3, 4 or 5 spleen tissue-specific genes, for which markers correspond to SEQ ID NO:s 41, 42, 43, 44 and/or 45; at least 1, 2, 3, 4 or 5 testis tissue-specific genes for which markers correspond to SEQ ID NO:s 46, 47, 48, 49 and/or 50; at least 1, 2, 3, 4 or 5 thyroid tissue-specific genes, for which markers correspond to SEQ ID NO:s 51, 52, 53, 54 and/or 55; or a combination thereof.

In another embodiment of the present invention, the method of the present invention provides that the tumor is chosen from the group comprising carcinoma, sarcoma, melanoma, central nervous system tumor, peripheral nerve tumor, soft tissue tumor and/or lymphoma tumor.

The present invention further provides in another embodiment that the expression level of the method of the present invention is determined at the nucleic acid level, and preferably using a microarray or quantitative PCR.

In yet another embodiment of the present invention the expression level of the method of the present invention is determined at the nucleic acid level using well known Next Generation Sequencing technologies, such as, for example, deep-sequencing technologies (Zhong et al. Nature Reviews Genetics 2009, 10, 57-63).

According to the method of the present invention, the microarray data is normalised prior to the classification. The normalisation of the microarray data can occur using normalisation methods known in the art. Typical normalisation methods include, but are not limited to global normalization, quantile normalization (RMA), Lowess or PLIER algorithm (Affymetrix).

In a preferred embodiment, the expression of all markers is assessed simultaneously by hybridization to a microarray. Preferably an Affymetrix platform is used for the measurement of the expression levels and more preferably an Affymetrix 133 Plus 2.0 gene expression array is used.

In yet another embodiment of the present invention, said plurality of tissue-specific genes comprises the tissue-specific genes for which markers are listed in Table 1.

In another embodiment of the present invention said plurality of tissue-specific genes consists of each of the genes for which markers are listed in Table 1.

In another embodiment, the present invention relates to a method according to the present invention, wherein the expression level is determined at the protein level. Determining the protein expression level in the method of the present invention enables the quantification of classifier proteins in tumor biopsies, in plasma, serum or on any type of tissue as mentioned in the present application. By measuring the expression level at the protein level it is possible to direct the measurement to very specific proteins and measure for instance shedded and/or secreted proteins. When using the protein expression level for the classification of tumors, the classifier as used in the present invention is based on the proteins encoded by the tissue-specific genes for which markers are listed in Table 1.

In another embodiment, the present invention provides a microarray comprising a plurality of probes complementary and hybridisable to sequences in at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55 or more different genes for which markers are listed in Table 1, wherein said plurality of probes is at least 50%, 60%, 70%, 80%, 90%, 95% or 100% of probes on said microarray.

In yet another embodiment, the present invention provides a microarray for determining the site of origin of a tumor, comprising a positionally-addressable array of a plurality of polynucleotide probes bound to a support, said plurality of polynucleotide probes comprising of different nucleotide sequences, each of said different nucleotide sequences comprising a sequence complementary and hybridisable to a sequence in a different gene, said plurality of polynucleotide probes consisting of different probes complementary and hybridisable to sequences in at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55 or more different genes selected from the group consisting of SEQ ID NO:s 1 to 55, wherein said plurality of polynucleotide probes on the microarray is at least 50%, 60%, 70%, 80%, 90%, 95% or 100% of probes on said microarray.

In another embodiment, the present invention provides a computer system comprising a processor, and a memory coupled to said processor and encoding one or more programs, wherein said one or more programs instruct the processor to carry out the method of the present invention.

In yet another embodiment, the present invention provides a computer program product for use in conjunction with a computer having a processor and a memory connected to the processor, said computer program product comprising a computer readable storage medium having a computer program mechanism encoded thereon, wherein said computer program mechanism may be loaded into the memory of said computer and cause said computer to carry out the method of the present invention.

In another embodiment, the present invention provides a kit for determining the site of origin of a tumor, comprising at least one microarray comprising probes to at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55 or more different tissue-specific genes for which markers are listed in Table 1, and a computer readable medium having recorded thereon one or more programs for carrying out the method of the present invention.

One skilled in this art will recognize that the above description is illustrative rather than exhaustive. Indeed, many additional formulations techniques and pharmaceutically-acceptable excipients and carrier solutions are well-known to those skilled in the art, as is the development of suitable dosing and treatment regimens for using the particular compositions described herein in a variety of treatment regimens.

The above aspects and embodiments are further supported by the following non-limiting examples.

The present examples demonstrate how the tissue specific markers are able to discriminate the different primary sites of CUPs.

### EXAMPLES

### EXAMPLE 1 - Selection of sets of tissue-specific genes

The present example demonstrates on how tissue specific probe sets were selected. Probe sets, 5 probe sets per tissue class, were selected for a total of 11 tissue classes. The 11 tissues classes were breast tissue, cerebellum tissue, heart tissue, kidney tissue, liver tissue, muscle tissue, pancreas tissue, prostate tissue, spleen tissue, testis tissue and thyroid tissue.

In a first step the expression levels of samples from the 11 different tissues were determined by performing an Affymetrix 133 Plus 2.0 whole genome array on biopsy material of the tissues. From the obtained results, a set of 100 housekeeping genes were re-scaled so the average values of these housekeeping genes was equal across all chips. The average was calculated for the three replicates of each tissue in order to create an average signal for each of the individual 11 tissue samples. An average value for the pooled 11 tissues was also calculated and used to create a Reference Tissue sample (RTS). The ratio was determined between each individual sample and the RTS.

The next step tissue-specific genes with a uniformly high expression in a specific tissue and a uniformly low expression among all other tissues were identified. A group of 55 probes were selected representing 11 sets of 5 probes for each tissue class. The genes represented by these probes are represented in Table 1.

Each set of 5 tissue-specific genes was considered representative for a specific tissue category.

### EXAMPLE 2 - Determining the origin of a tissue sample

The present example illustrates on how the 11 sets of 5 tissue-specific genes can be used to specifically determine the origin of a tissue sample.

RNA samples (commercially available http://www.affymetrix.com/support/technical/ sample_data/exon_array_data.affx) from 11 different tissue samples (in triplicate) corresponding to each of the specific tissue categories were analyzed using the Affymetrix 133 Plus 2.0 gene expression microarray.

To classify the obtained results, the ratio was determined for each set of 5 tissue-specific genes (pooled) and the RTS. This average ratio constitutes the score for that specific tissue category. The scores of the 33 tissue samples are provided in Table 3.

From the table it is clear that each set of the 5 selected tissue-specific genes can unambiguously determine the correct tissue represented by the respective gene category.

### EXAMPLE 3 - Determining the origin of a mixed tissue sample

Experiments similar to the experiments of Example 2 were performed on samples containing a mix of two or three tissues according to Table 4

Table 5 shows the results of the 4 mixtures represented in Table 4. From the table it is clear that the tissues that were used to produce the mixture can be determined unambiguously and the semi-quantitative nature of the score is also obvious form the scores. Mixture 3 contained 33% of heart tissue, testis tissue and cerebellum tissue. The scores obtained by the method of the invention are approximately 4 for heart tissue, approximately 5 for testis tissue and approximately 5 for cerebellum tissue. Mixture 4 consisted of 33% heart tissue, 17% testis tissue and 50% cerebellum tissue. Accordingly, the scores for heart tissue were still approximately 4 but the score for testis tissue were approximately 3 and the scores for cerebellum tissue were approximately 7, clearly indicating the shift in tissue composition of the sample.

These results show the quantitative character of the scores, and show that it is possible to estimate tissue compositions in this manner.

### EXAMPLE 4 - Assessment of the reproducibility and robustness of the method

To further demonstrate the sensitivity of the 11 sets of 5 tissue-specific genes spike-in experiments with the 55 tissue-specific genes were carried out. For these experiments a dilution series experiment was performed (according to Barnes, M. et al. Nucl. Acids Res. 2005, 33, pp. 5914-5923). By mixing two different RNA samples at known proportions and analyzing these samples in duplo this type of experimental design is able to compare two microarray platforms. The same technique is used here to demonstrate the quantitative nature of the score in relation to the level of contamination in the sample. For the present example a Peripheral Blood Mononuclear Cell (PBMC) sample is diluted with placenta RNA.

Table 6 shows the results of individual gene levels and it demonstrates that individual gene levels can be assessed when these genes are relevant for targeted decision making. From the table it is clear that the score for B cells, T cells, NK cells, monocytes and neutrophiles drop significantly as the PBMC sample is diluted with placenta RNA. On the other hand, the score for dentritic cells and macrophages is low as these cells are not present in circulating leukocytes. The scores for connective tissue gene categories like endothelial cells, lymphatic cells, fibroblasts and extracellular matrix scores increase significantly as the level of placenta RNA increases.

It is also clear that for the individual hematopoietic genes with therapeutic implications like CD20 (target for Rituxan) and CD52 (target for alemtuzumab) decrease according to the PBMC dilution with placenta RNA, while the non-hematopoietic targets like PDGFRA (target for imatinib/Gleevec) and EGFR (target for gefitinib, erlotinib) increase significant with increasing placenta RNA levels).

Altogether, these results provide additional evidence for the capability of the classification method to provide information about tissue constitution. Furthermore the replicate results shown in Tables 3, 5 and 6 show that the technical replicates in the data sets used give very similar results providing evidence for the good reproducibility and robustness of the method.

### EXAMPLE 5 - Assessment of the reproducibility and robustness of the method using tumor tissue

The present example shows further the reproducibility and robustness of the method of the present invention. Several laboratories performed a microarray experiment on the same sample. Five replicates were analyzed of the commercial Universal Human Reference RNA by six independent laboratories. Stratagene's Universal Human Reference RNA is composed of total RNA from 10 human cell lines (mammary gland adenocarcinoma, liver hepatoblastoma, cervix adenocarcinoma, testis embryonal carcinoma, brain glioblastoma, melanoma, liposarcoma, histiocytic lymphoma, lymphoblastic leukaemia and plasmacytoma. The Universal Reference RNA is designed to be used as a reference for microarray gene-profiling experiments.

Table 7 shows that the correlation between the technical replicates is in the range between 0.95 and 0.99 for the majority of the comparisons showing the robustness of the scores. Furthermore, these results show that Stratagene's Universal Reference RNA can also be used to monitor the performance of a laboratory that uses the method of the invention. Scores produced by any laboratory that wants to use the method of the invention as a diagnostic tool should produce scores for the Reference RNA very similar to those in Table 7.

### EXAMPLE 6 - Determining the primary site of tumor tissue samples

To further evaluate the accuracy of the 11 sets of 5 tissue-specific genes in their ability to determine the site of origin of tumors of unknown primary, a large dataset containing more that 2000 tumor samples was used. The microarray data of this dataset has been submitted to GEO (accession GSE2109). The classification efficiency of the 11 sets of 5 tissue-specific genes was tested with the most recent submission. This submission was not used to train the classifier. Batch 16 of this data set became public on 31 December 2008. The primary site of origin of 60 out of the 62 tumors in this batch were correctly determined using the 11 sets of 5 tissue-specific genes.

In another study a large set of 225 tumor samples from the MD Anderson Comprehensive Cancer Center of which the gene expression microarray data were provided in a blinded fashion. The method of the present invention enabled to correctly determine primary site of 221 tumor samples (98% correct).

The method of the present invention therefore enables the accurate, robust and fast classification of a tumor sample and the determination of the primary site of the tumors.

**Table 1: List of 55 tissue-specific marker sequences**

| **SEQ ID NO** | **Gene name** | **Gene Symbol** | **Nucleotide Sequence** | **Tissue** |
|---|---|---|---|---|
| 1 | perilipin | PLIN | TCCAGGCCTGTGTGCTTTGTAGAGC | breast |
| 2 | secretoglobin, family 2A, member 2 | SCGB2A2 | GCAGCAGCCTCACCATGAAGTTGCT | breast |
| 3 | prolactin-induced protein | PIP | GGGGGCCAACAAAGCTCAGGACAAC | breast |
| 4 | secretoglobin, family ID, member 2 | SCGB1D2 | TAGAAGTCCAAATCACTCATTGTTT | breast |
| 5 | keratin 14 (epidermolysis bullosa simplex, Dowling-Meara, Koebner) | KRT14 | GGATCGCAGTCATCCAGAGATGTGA | breast |
| 6 | synaptosomal-associated protein, 25kDa | SNAP25 | GCATGCTCAGTATTGAGACACTGTC | cerebellum |
| 7 | glial fibrillary acidic protein | GFAP | CTGCTTCTTAACCCCAGTAAGCCAC | cerebellum |
| 8 | glutamate receptor, ionotropic, AMPA 2 | GRIA2 | ATCTTCCTCGCAGAATTCACAGAAT | cerebellum |
| 9 | synuclein, beta | SNCB | ACCAAGGAACAGGCCTCACATCTGG | cerebellum |
| 10 | gamma-aminobutyric acid (GABA) A receptor, delta | GABRD | GCAGCTGCCCAGAAACTTCCTGGGA | cerebellum |
| 11 | troponin I type 3 (cardiac) | TNNI3 | AAAATCTAAGATCTCCGCCTCGAGA | heart |
| 12 | natriuretic peptide precursor B | NPPB | GTTCAGCCTCGGACTTGGAAACGTC | heart |
| 13 | myosin binding protein C, cardiac | MYBPC3 | CCTGGACCTGGGAGAAGACGCCCGC | heart |
| 14 | troponin T type 2 (cardiac) | TNNT2 | CGGCAGAACCGCCTGGCTGAAGAGA | heart |
| 15 | myosin, light chain 7, regulatory | MYL7 | AAGAAGCCTTCAGCTGTATCGACCA | heart |
| 16 | chloride channel Kb | CLCNKB | TGTCAAGAAGCTGCCATACCTGCCA | kidney |
| 17 | cadherin 16, KSP-cadherin | CDH16 | GAACACATAATCCCCGTGGTGGTCA | kidney |
| 18 | uromodulin (uromucoid, Tamm-Horsfall glycoprotein) | UMOD | GATTTTCCGTCCAGATGTTCCGGTT | kidney |
| 19 | solute carrier family 12 (sodium/chloride transporters), member 3 | SLC12A3 | GGCTCTTTGACGATGGAGGCCTCAC | kidney |
| 20 | potassium inwardly-rectifying channel, subfamily J, member 1 | KCNJ1 | AACAATTTGAGGCTCTAAGCTTCTC | kidney |
| 21 | fibrinogen alpha chain | FGA | TCACTGAATCTAACCATAGCTGACC | liver |
| 22 | C-reactive protein, pentraxin-related | CRP | AGCGCTGATCTTCTATTTAATTCCC | liver |
| 23 | hemopexin | HPX | GGGAGGCTATACCCTAGTAAGCGGT | liver |
| 24 | fibrinogen beta chain | FGB | GGTCATCGACCCCTTGACAAGAAGA | liver |
| 25 | apolipoprotein A-II | APOA2 | GCACAGACACCAAGGACAGAGACGC | liver |
| 26 | fast skeletal myosin light chain 2 | HUMMLC2B | TCTCCATGTTCGACCAGACTCAGAT | muscle |
| 27 | myosin binding protein C, fast type | MYBPC2 | GGCACACTAGCTGTACTGTGTCCGA | muscle |
| 28 | calcium channel, voltage-dependent, gamma subunit 1 | CACNG1 | GAACCCATGGGAGTCCTGCATGGAT | muscle |
| 29 | fructose-1,6-bisphosphatase 2 | FBP2 | CGGCCACCACTGAATATGTGCAGAA | muscle |
| 30 | synaptopodin 2 | SYNP02 | CTGGGATTCTGGACTGGTGGACATT | muscle |
| 31 | carboxypeptidase A1 (pancreatic) | CPA1 | CTGGCTTTGGGTTGTCCGGAGCCAG | pancreas |
| 32 | protease, serine, 1 (trypsin 1) | PRSS1 | CCACCCCCAATACGACAGGAAGACT | pancreas |
| 33 | pancreatic lipase | PNLIP | GATAGCATCGTCAACCCTGATGGCT | pancreas |
| 34 | chymotrypsinogen B1 /// chymotrypsinogen B2 /// similar to Chymotrypsinogen B precursor | CTRB1 | GACCAAGTACAACGCCAACAAGACC | pancreas |
| 35 | insulin | INS | GAAGAGGCCATCAAGCACATCACTG | pancreas |
| 36 | kallikrein-related peptidase 3 | KLK3 | TGGTGTAATTTTGTCCTCTCTGTGT | prostate |
| 37 | microseminoprotein, beta- | MSMB | GTACCTGTCTATAAGGAGTCCTGCT | prostate |
| 38 | homeobox B13 | HOXB13 | TTGCCTTCTATCCGGGATATCCGGG | prostate |
| 39 | kallikrein-related peptidase 2 | KLK2 | CTACTGACCTGTGCTTTCTGGTGTG | prostate |
| 40 | chloride channel, calcium activated, family member 4 | CLCA4 | AGTAACTTTGTTTATCCCTCAAGCA | prostrate |
| 41 | CD37 molecule | CD37 | TACCCGCAGGACTGGTTCCAAGTCC | spleen |
| 42 | chemokine (C-X-C motif) ligand 13 (B-cell chemoattractant) | CXCL13 | GGAGTTTGCATTCTTATTCATCAGG | spleen |
| 43 | Fc fragment of IgE, low affinity II, receptor for (CD23) | FCER2 | TTGAGCATGGATACAGCCAGGCCCA | spleen |
| 44 | membrane-spanning 4-domains, subfamily A, member 1 | MS4A1 | GAACCTCCCCAAGATCAGGAATCCT | spleen |
| 45 | immunoglobulin heavy constant delta | IGHD | TCTACAGCGGCATTGTCACTTTCAT | spleen |
| 46 | protamine 1 | PRM1 | GCTGACAGGTTGGCTGGCTCAGCCA | testis |
| 47 | sperm mitochondria-associated cysteine-rich protein | MCSP | AAGGCAGTCAATGCTGCCCACCAAA | testis |
| 48 | transition protein 1 (during histone to protamine replacement) | TNP1 | AAGAAAATACCATGTCGACCAGCCG | testis |
| 49 | protamine 2 | PRM2 | GAGCGAACGCTCGCACGAGGTGTAC | testis |
| 50 | germ cell associated 1 | GSG1 | GTGGGCTCAAACTGAGCGCCTTTGC | testis |
| 51 | thyroglobulin | TG | AAAACTACGGCCATGGCAGCCTGGA | thyroid |
| 52 | parathyroid hormone | PTH | AATACAGCTTATGCATAACCTGGGA | thyroid |
| 53 | thyroid stimulating hormone receptor | TSHR | AGCCCTGTTGATCACTGGACATAAA | thyroid |
| 54 | thyroid peroxidase | TPO | TGAACGAGTGTGCAGACGGTGCCCA | thyroid |
| 55 | NK2 homeobox 1 | TITF1 | GTGATTCAAATGGGTTTTCCACGCT | thyroid |

**Table 2: List of tissues and their respective tumor subclasses**

| **Tissue** | **Tumor type** | **Tissue** | **Tumor type** |
|---|---|---|---|
| Adrenal gland | Cortical carcinoma | Larynx | Adenocarcinoma |
| Adrenal gland | Malignant medullary tumor | Larynx | Squamous cell carcinoma |
| Adrenal gland | Subcapsular cell carcinoma | Liver | Cholangiocarcinoma |
| Bone marrow | Myelodysplastic hematopoietic disorder | Liver | Hepatocellular carcinoma |
| Brain | Astrocytoma, malignant | Lung | Adenocarcinoma |
| Brain | Choroid plexus carcinoma | Lung | Adenosquamous carcinoma, |
| Brain | Glioblastoma | Lung | Bronchiolo carcinoma, |
| Brain | Glioma, anaplastic | Lung | Mucoepidermoid carcinoma, |
| Brain | Glioma, astrocytic, malignant | Lung | Squamous cell carcinoma |
| Brain | Glioma, oligodendrocytic, malignant | Mammary gland | Adenocarcinoma |
| Brain | Malignant astrocytoma | Nasal cavity | Adenocarcinoma |
| Brain | Malignant ependymoma | Nasal cavity | Adenosquamous carcinoma |
| Brain | Malignant mixed glioma | Nasal cavity | Neuroblastoma, olfactory |
| Brain | Malignant oligodendroglioma | Nasal cavity | Squamous cell carcinoma |
| Bronchiole | Adenocarcinoma | Nasopharynx | Adenocarcinoma |
| Bronchiole | Squamous cell carcinoma | Nasopharynx | Neuroblastoma, olfactory |
| Bronchus | Squamous cell carcinoma | Nasopharynx | Squamous cell carcinoma |
| Bulbourethral gland | Adenocarcinoma | Oral cavity | Adenocarcinoma |
| Cecum | Adenocarcinoma | Oral cavity | Squamous cell carcinoma |
| Cerebellum | Medulloblastoma | Ovary | Adenocarcinoma, tubulostromal |
| Cerebral meninx | Malignant meningioma | Ovary | Embryonal carcinoma, |
| Cerebral meninx | Sarcoma, meningeal | Ovary | Yolk sac Carcinoma, |
| Colon | Adenocarcinoma | Ovary | Choriocarcinoma |
| Duodenum | Adenocarcinoma | Ovary | Chorioepithelioma, malignant |
| Epididymis | Adenoma, Leydig cell | Ovary | Granulosa cell tumor, malignant |
| Esophagus | Squamous cell carcinoma | Ovary | Teratoma, malignant |
| Eyeball | Uveal melanoma, malignant | Ovary | Tumor, Sertoli cell, malignant |
| Gallbladder | Adenocarcinoma | Ovary | Tumor, theca cell, malignant |
| Glandular stomach | Adenocarcinoma | Oviduct | Embryonal carcinoma |
| Glandular stomach | Malignant neuroendocrine cell tumor | Oviduct | Yolk sac Carcinoma, |
| Harderian gland | Adenocarcinoma | Oviduct | Choriocarcinoma |
| Hematopoietic tissue | Erythroid leukemia | Pancreas | Adenocarcinoma |
| Hematopoietic tissue | Granulocytic leukemia | Pancreas | Adenocarcinoma, acinar cell |
| Hematopoietic tissue | Myeloid Leukemia | Pancreas | Adenocarcinoma, endocrine pancreas |
| Hematopoietic tissue | Lymphoma | Paranasal sinus | Adenocarcinoma |
| Hematopoietic tissue | Malignant mast cell tumor | Paranasal sinus | Neuroblastoma, olfactory |
| Hematopoietic tissue | Megakaryocytic leukemia | Paranasal sinus | Squamous cell carcinoma |
| Ileum | Adenocarcinoma | Parathyroid gland | Adenoarcinoma |
| Jejunum | Adenocarcinoma | Parotid gland | Adenocarcinoma |
| Joint | Synovial sarcoma | Parotid gland | Malignant mixed tumor |
| Kidney | Renal adenocarcinoma | Pineal gland | Pinealoma, malignant |
| Kidney | Sarcoma, renal | Pituitary gland | Carcinoma, pars distalis |
| Kidney | Wilms' tumor | Pituitary gland | Carcinoma, pars intermedia |
| Large intestine | Adenocarcinoma | Pleura | Malignant mesothelioma |
| Preputial gland | Adenocarcinoma, acinar cell | Testis | Sertoli cell carcinoma |
| Preputial gland | Squamous cell carcinoma | Thymus | Malignant thymoma |
| Prostate | Adenocarcinoma | Thyroid gland | Adenocarcinoma |
| Rectum | Adenocarcinoma | Thyroid gland | Parafollicular cell carcinoma |
| Renal pelvis | Squamous cell carcinoma | Thyroid gland | Follicular cell carcinoma |
| Renal pelvis | Transitional cell carcinoma, | Tongue | Adenocarcinoma |
| Renal pelvis | Urothelial carcinoma, | Tongue | Squamous cell carcinoma |
| Salivary gland | Adenocarcinoma | Tooth | Odontoma |
| Salivary gland | Mixed tumor, malignant | Trachea | Adenocarcinoma |
| Salivary gland | Myoepithelioma, malignant | Trachea | Squamous cell carcinoma |
| Seminal vesicle | Adenocarcinoma | Ureter | Squamous cell carcinoma |
| Seminal vesicle | Adenosquamous carcinoma | Ureter | Transitional cell carcinoma |
| Seminal vesicle | Leiomyosarcoma | Ureter | Urothelial carcinoma |
| Skeletal muscle | Rhabdomyosarcoma | Urethral gland | Adenocarcinoma |
| Skeletal system | Chondrosarcoma | Urinary bladder | Squamous cell carcinoma |
| Skeletal system | Osteosarcoma | Urinary bladder | Transitional cell carcinoma |
| Skin/subcutaneous tissue | Basal cell Carcinoma, | Urinary bladder | Urothelial carcinoma |
| Skin/subcutaneous tissue | Sebaceous cell Carcinoma | Uterine cervix | Adenocarcinoma |
| Skin/subcutaneous tissue | Squamous cell carcinoma | Uterine cervix | Squamous cell carcinoma |
| Skin/subcutaneous tissue | Malignant melanoma | Uterus | Adenocarcinoma |
| Small intestine | Adenocarcinoma | Uterus | Sarcoma, endometrial stromal |
| Soft tissue | Fibrosarcoma | Uterus | Leiomyosarcoma |
| Soft tissue | Fibrous histiocytoma, malignant | Vagina | Squamous cell carcinoma |
| Soft tissue | Hemangiosarcoma | Zymbal gland | Squamous cell carcinoma |
| Soft tissue | Leiomyosarcoma | Zymbal gland | Sebaceous cell Carcinoma |
| Soft tissue | Liposarcoma | Testis | Leydig cell carcinoma |
| Soft tissue | Sarcoma, NOS | Testis | Yolk sac carcinoma |
| Soft tissue | Schwannoma, malignant | Testis | Germinoma, malignant |
| Spinal cord | Astrocytoma, malignant | Testis | Granulosa cell tumor |
| Spinal cord | Glioblastoma | Testis | Malignant seminoma |
| Spinal cord | Glioma, anaplastic | Testis | Malignant Sertoli cell tumor |
| Spinal cord | Glioma, astrocytic, malignant | Testis | Malignant teratoma |
| Spinal cord | Glioma, oligodendrocytic, malignant | Testis | Rete testis carcinoma |
| Spinal cord | Oligoastroglioma, malignant | Testis | Teratoma, malignant |
| Spinal meninx | Meningioma, malignant | | |
| Sublingual gland | Adenocarcinoma | | |
| Sublingual gland | Mixed tumor, malignant | | |
| Sublingual gland | Myoepithelioma, malignant | | |

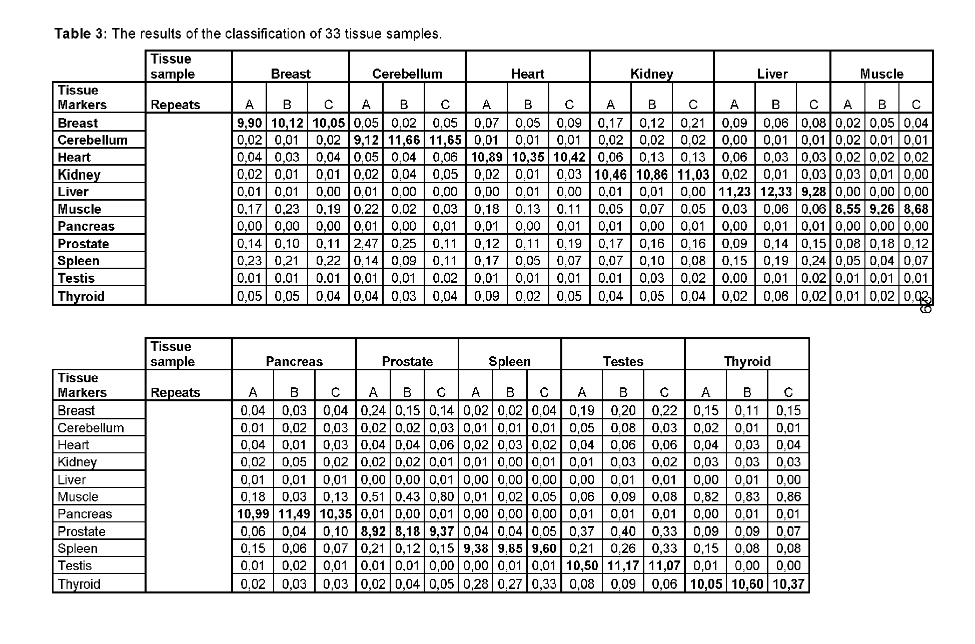

**Table 4: Overview of four samples wherein two or three tissues are mixed in a specific proportion.**

| | Mix 1 | Mix 2 | Mix 3 | Mix 4 |
|---|---|---|---|---|
| Heart | 0 | 0 | 0.33 | 0.33 |
| Testes | 0.5 | 0.33 | 0.33 | 0.17 |
| Cerebellum | 0.5 | 0.67 | 0.33 | 0.5 |

**Table 5: The classification results of the 4 mixtures represented in Table 4.**

| | **Mix** | **Mix 1** | | | | | **Mix 2** | | | | | **Mix 3** | | | | | **Mix 4** | | | | |
|---|---|---|---|---|---|---|---|---|---|---|---|---|---|---|---|---|---|---|---|---|---|
| **Tissue markers** | **Repeat** | A | B | C | D | E | A | B | C | D | E | A | B | C | D | E | A | B | C | D | E |
| Breast | | 0,08 | 0,15 | 0,13 | 0,17 | 0,09 | 0,10 | 0,08 | 0,16 | 0,13 | 0,12 | 0,11 | 0,15 | 0,19 | 0,12 | 0,13 | 0,06 | 0,10 | 0,06 | 0,06 | 0,04 |
| Cerebellum | | **5,77** | **6,01** | **5,98** | **6,09** | **6,09** | **7,44** | **7,95** | **7,91** | **8,03** | **7,91** | **4,92** | **4,87** | **5,16** | **5,16** | **4,95** | **6,90** | **7,00** | **7,01** | **6,72** | **6,77** |
| Heart | | 0,10 | 0,11 | 0,07 | 0,09 | 0,11 | 0,09 | 0,05 | 0,09 | 0,13 | 0,05 | **3,86** | **3,75** | **3,81** | **4,04** | **4,10** | **3,90** | **4,05** | **3,93** | **4,02** | **4,08** |
| Kidney | | 0,02 | 0,05 | 0,02 | 0,02 | 0,02 | 0,02 | 0,01 | 0,06 | 0,02 | 0,05 | 0,03 | 0,02 | 0,03 | 0,03 | 0,02 | 0,02 | 0,02 | 0,02 | 0,02 | 0,03 |
| Liver | | 0,01 | 0,00 | 0,01 | 0,01 | 0,01 | 0,01 | 0,01 | 0,00 | 0,01 | 0,01 | 0,01 | 0,01 | 0,00 | 0,00 | 0,01 | 0,01 | 0,00 | 0,01 | 0,00 | 0,01 |
| Muscle | | 0,13 | 0,08 | 0,07 | 0,04 | 0,11 | 0,08 | 0,10 | 0,05 | 0,10 | 0,07 | 0,08 | 0,08 | 0,08 | 0,18 | 0,14 | 0,05 | 0,07 | 0,10 | 0,09 | 0,10 |
| Pancreas | | 0,01 | 0,01 | 0,00 | 0,01 | 0,01 | 0,01 | 0,00 | 0,01 | 0,01 | 0,01 | 0,00 | 0,01 | 0,00 | 0,01 | 0,01 | 0,01 | 0,00 | 0,00 | 0,00 | 0,00 |
| Prostate | | 0,39 | 0,15 | 0,24 | 0,21 | 0,23 | 0,22 | 0,16 | 0,21 | 0,14 | 0,24 | 0,26 | 0,13 | 0,27 | 0,29 | 0,19 | 0,20 | 0,37 | 0,14 | 0,27 | 0,34 |
| Spleen | | 0,16 | 0,08 | 0,26 | 0,20 | 0,23 | 0,12 | 0,13 | 0,14 | 0,07 | 0,47 | 0,20 | 0,12 | 0,11 | 0,12 | 0,10 | 0,09 | 0,09 | 0,10 | 0,12 | 0,15 |
| Testis | | **7,31** | **7,58** | **7,14** | **7,46** | **7,20** | **5,05** | **5,52** | **5,42** | **5,64** | **5,36** | **5,82** | **5,65** | **5,26** | **5,53** | **5,19** | **3,40** | **3,20** | **3,33** | **3,32** | **3,60** |
| Thyroid | | 0,06 | 0,05 | 0,04 | 0,05 | 0,04 | 0,04 | 0,05 | 0,08 | 0,08 | 0,02 | 0,03 | 0,15 | 0,06 | 0,11 | 0,15 | 0,07 | 0,04 | 0,03 | 0,03 | 0,03 |

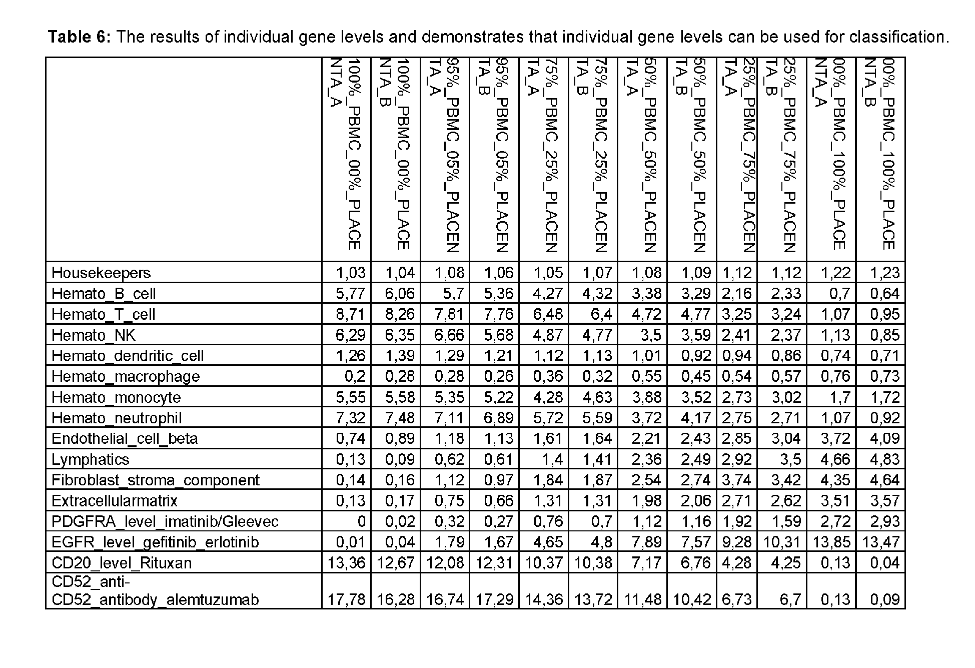

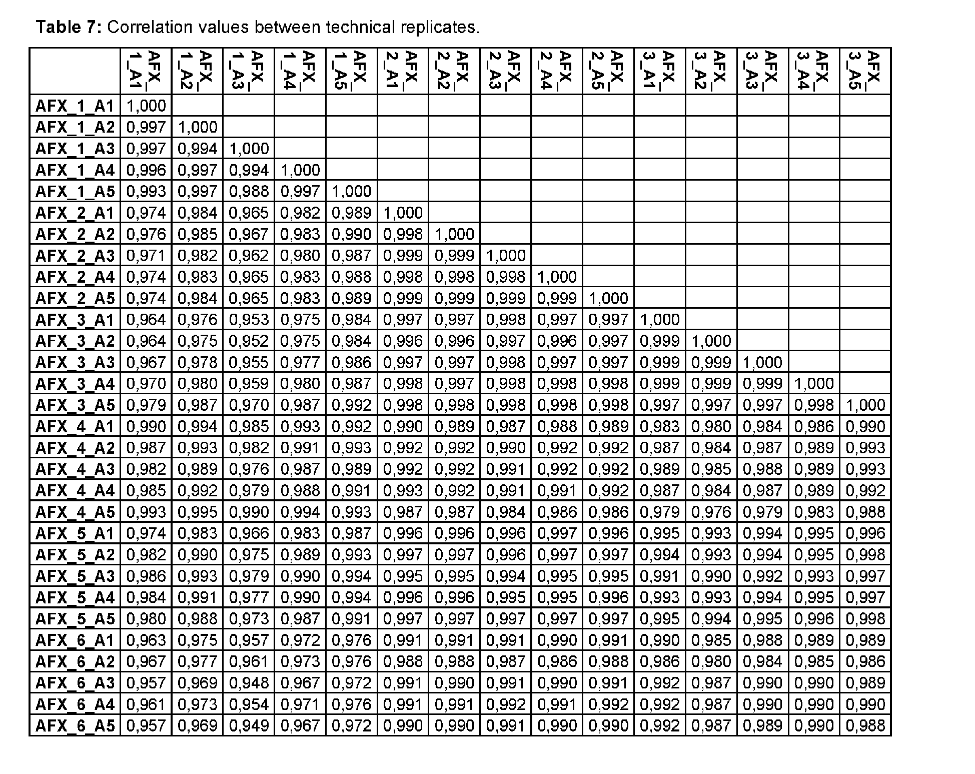

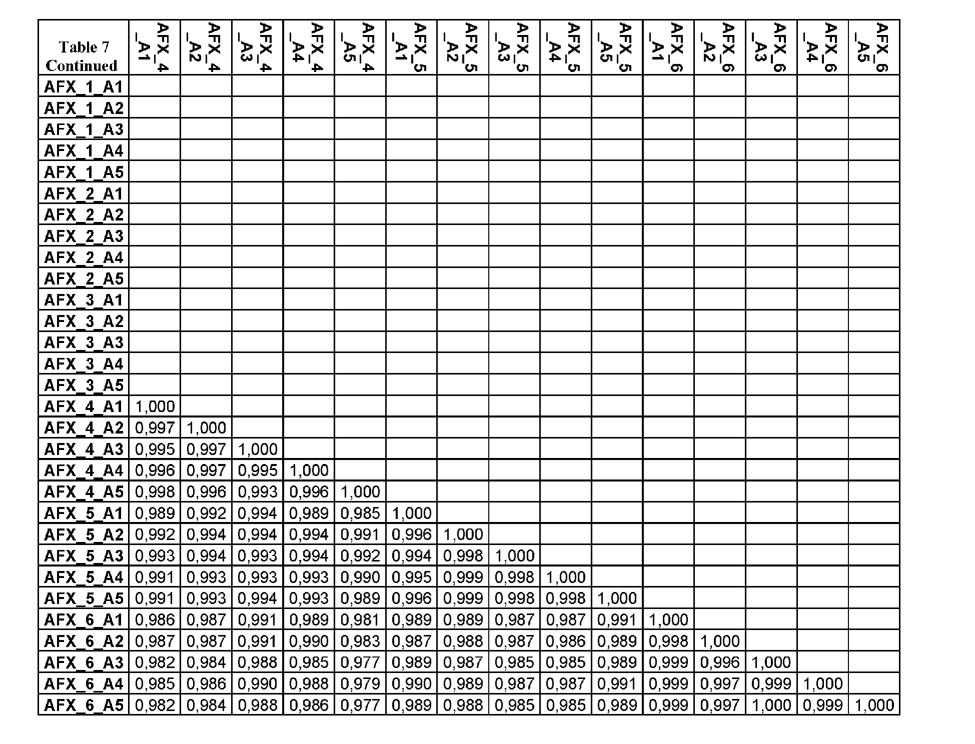

## Claims

1. A method for classifying a tumor according to the site of origin of said tumor, comprising:
(a) determining the expression profile of a sample;
(b) calculating a classifier parameter between said expression profile and a tissue-specific template;
said expression profile comprising the expression levels of a plurality of tissue-specific genes in said sample;
said plurality of tissue-specific genes consisting of at least 1 of the tissue-specific genes for which markers are listed in Table 1;
said tissue-specific template comprising, for each tissue-specific gene in said plurality of tissue-specific genes, the representative expression level of said tissue-specific gene in said tissue;
(c) classifying said tumor according to the site of origin if said classifier parameter is above a chosen threshold or if said expression profile is more similar to a tissue-specific template than to another tissue-specific template.

2. The method according to Claim 1, wherein step (b) is repeated for a plurality of tissue-specific templates, each tissue-specific template being representative for a specific tissue, thereby calculating a plurality of classifier parameters.

3. The method according to Claims 1 or 2, wherein the method additionally comprises the steps of:
(a) isolating nucleic acids from a sample; and
(b) determining the expression levels of a plurality of tissue-specific genes in said isolated nucleic acids.

4. The method according to any of Claims 1 to 3, wherein a plurality of classifier parameters are calculated.

5. The method according to any of Claims 1 to 4, wherein SEQ ID NO:s 1 to 5 are representative for breast tissue, SEQ ID NO:s 6 to 10 are representative for cerebellum tissue, SEQ ID NO:s 11 to 15 are representative for heart tissue, SEQ ID NO:s 16 to 20 are representative for kidney tissue, SEQ ID NO:s 21 to 25 are representative for liver tissue, SEQ ID NO:s 26 to 30 are representative for muscle tissue, SEQ ID NO:s 31 to 35 are representative for pancreas tissue, SEQ ID NO:s 36 to 40 are representative for prostate tissue, SEQ ID NO:s 41 to 45 are representative for spleen tissue, SEQ ID NO:s 46 to 50 are representative for testis tissue, and SEQ ID NO:s 51 to 55 are representative for thyroid tissue.

6. The method according any of Claims 1 to 5, wherein said tumor is chosen from the group comprising carcinoma, sarcoma, melanoma and/or lymphoma tumor.

7. The method according any of Claims 1 to 6, wherein said expression level is determined at the nucleic acid level.

8. The method according any of Claims 1 to 7, wherein said expression level is determined using a microarray.

9. The method according any of Claims 1 to 7, wherein said expression level is determined using next generation sequencing techniques.

10. The method according any of Claims 1 to 7, wherein said expression level is determined using quantitative PCR.

11. The method according any of Claims 1 to 9, wherein said plurality of tissue-specific genes comprises the tissue-specific genes for which markers are listed in Table 1.

12. The method according any of Claims 1 to 9, wherein said plurality of tissue-specific genes consists of each of the genes for which markers are listed in Table 1.

13. The method according any of Claims 1 to 6, wherein said expression level is determined at the protein level.

14. A microarray comprising a plurality of probes complementary and hybridisable to sequences in at least 1 different genes for which markers are listed in Table 1, wherein said plurality of probes is at least 50% of probes on said microarray.

15. A computer system comprising a processor, and a memory coupled to said processor and encoding one or more programs, wherein said one or more programs instruct the processor to carry out the method of any one of claims 1 to 12.

16. A computer program product for use in conjunction with a computer having a processor and a memory connected to the processor, said computer program product comprising a computer readable storage medium having a computer program mechanism encoded thereon, wherein said computer program mechanism may be loaded into the memory of said computer and cause said computer to carry out the method of any one of claims 1 to 12.

17. A kit for determining the site of origin of a tumor, comprising at least one microarray comprising probes to at least 1 different tissue-specific genes for which markers are listed in Table 1, and a computer readable medium having recorded thereon one or more programs for carrying out the method of any one of claims 1 to 13.


sequence Listing

<110>  UNIVERSITEIT MAASTRICHT
       ACADEMISCH ZIEKENHUIS MAASTRICHT

<120>  METHOD FOR DETERMINING THE PRIMARY SITE OF CUP

<130>  UMA-011-EPPRIO

<160>  55

<170>  PatentIn version 3.3

<210>  1
<211>  25
<212>  DNA
<213>  Homo sapiens

<400>  1
tccaggcctg tgtgctttgt agagc                                           25


<210>  2
<211>  25
<212>  DNA
<213>  Homo sapiens

<400>  2
gcagcagcct caccatgaag ttgct                                           25


<210>  3
<211>  25
<212>  DNA
<213>  Homo sapiens

<400>  3
gggggccaac aaagctcagg acaac                                           25


<210>  4
<211>  25
<212>  DNA
<213>  Homo sapiens

<400>  4
tagaagtcca aatcactcat tgttt                                           25


<210>  5
<211>  25
<212>  DNA
<213>  Homo sapiens

<400>  5
ggatcgcagt catccagaga tgtga                                           25


<210>  6
<211>  25
<212>  DNA
<213>  Homo sapiens

<400>  6
gcatgctcag tattgagaca ctgtc                                           25


<210>  7
<211>  25

<212>  DNA
<213>  Homo sapiens

<400>  7
ctgcttctta accccagtaa gccac                                          25


<210>  8
<211>  25
<212>  DNA
<213>  Homo sapiens

<400>  8
atcttcctcg cagaattcac agaat                                          25


<210>  9
<211>  25
<212>  DNA
<213>  Homo sapiens

<400>  9
accaaggaac aggcctcaca tctgg                                          25


<210>  10
<211>  25
<212>  DNA
<213>  Homo sapiens

<400>  10
gcagctgccc agaaacttcc tggga                                          25


<210>  11
<211>  25
<212>  DNA
<213>  Homo sapiens

<400>  11
aaaatctaag atctccgcct cgaga                                          25


<210>  12
<211>  25
<212>  DNA
<213>  Homo sapiens

<400>  12
gttcagcctc ggacttggaa acgtc                                          25


<210>  13
<211>  25
<212>  DNA
<213>  Homo sapiens

<400>  13
cctggacctg ggagaagacg cccgc                                          25


<210>  14
<211>  25
<212>  DNA
<213>  Homo sapiens

<400>  14
cggcagaacc gcctggctga agaga                                          25

<210>  15
<211>  25
<212>  DNA
<213>  Homo sapiens

<400>  15
aagaagcctt cagctgtatc gacca                                          25


<210>  16
<211>  25
<212>  DNA
<213>  Homo sapiens

<400>  16
tgtcaagaag ctgccatacc tgcca                                          25


<210>  17
<211>  25
<212>  DNA
<213>  Homo sapiens

<400>  17
gaacacataa tccccgtggt ggtca                                          25


<210>  18
<211>  25
<212>  DNA
<213>  Homo sapiens

<400>  18
gattttccgt ccagatgttc cggtt                                          25


<210>  19
<211>  25
<212>  DNA
<213>  Homo sapiens

<400>  19
ggctctttga cgatggaggc ctcac                                          25


<210>  20
<211>  25
<212>  DNA
<213>  Homo sapiens

<400>  20
aacaatttga ggctctaagc ttctc                                          25


<210>  21
<211>  25
<212>  DNA
<213>  Homo sapiens

<400>  21
tcactgaatc taaccatagc tgacc                                          25


<210>  22
<211>  25
<212>  DNA
<213>  Homo sapiens

<400>  22
agcgctgatc ttctatttaa ttccc                                          25


<210>  23
<211>  25
<212>  DNA
<213>  Homo sapiens

<400>  23
gggaggctat accctagtaa gcggt                                          25


<210>  24
<211>  25
<212>  DNA
<213>  Homo sapiens

<400>  24
ggtcatcgac cccttgacaa gaaga                                          25


<210>  25
<211>  25
<212>  DNA
<213>  Homo sapiens

<400>  25
gcacagacac caaggacaga gacgc                                          25


<210>  26
<211>  25
<212>  DNA
<213>  Homo sapiens

<400>  26
tctccatgtt cgaccagact cagat                                          25


<210>  27
<211>  25
<212>  DNA
<213>  Homo sapiens

<400>  27
ggcacactag ctgtactgtg tccga                                          25


<210>  28
<211>  25
<212>  DNA
<213>  Homo sapiens

<400>  28
gaacccatgg gagtcctgca tggat                                          25


<210>  29
<211>  25
<212>  DNA
<213>  Homo sapiens

<400>  29
cggccaccac tgaatatgtg cagaa                                          25


<210>  30
<211>  25

<212>  DNA
<213>  Homo sapiens

<400>  30
ctgggattct ggactggtgg acatt                                          25


<210>  31
<211>  25
<212>  DNA
<213>  Homo sapiens

<400>  31
ctggctttgg gttgtccgga gccag                                          25


<210>  32
<211>  25
<212>  DNA
<213>  Homo sapiens

<400>  32
ccacccccaa tacgacagga agact                                          25


<210>  33
<211>  25
<212>  DNA
<213>  Homo sapiens

<400>  33
gatagcatcg tcaaccctga tggct                                          25


<210>  34
<211>  25
<212>  DNA
<213>  Homo sapiens

<400>  34
gaccaagtac aacgccaaca agacc                                          25


<210>  35
<211>  25
<212>  DNA
<213>  Homo sapiens

<400>  35
gaagaggcca tcaagcacat cactg                                          25


<210>  36
<211>  25
<212>  DNA
<213>  Homo sapiens

<400>  36
tggtgtaatt ttgtcctctc tgtgt                                          25


<210>  37
<211>  25
<212>  DNA
<213>  Homo sapiens

<400>  37
gtacctgtct ataaggagtc ctgct                                          25

<210>  38
<211>  25
<212>  DNA
<213>  Homo sapiens

<400>  38
ttgccttcta tccgggatat ccggg                                          25


<210>  39
<211>  25
<212>  DNA
<213>  Homo sapiens

<400>  39
ctactgacct gtgctttctg gtgtg                                          25


<210>  40
<211>  25
<212>  DNA
<213>  Homo sapiens

<400>  40
agtaactttg tttatccctc aagca                                          25


<210>  41
<211>  25
<212>  DNA
<213>  Homo sapiens

<400>  41
tacccgcagg actggttcca agtcc                                          25


<210>  42
<211>  25
<212>  DNA
<213>  Homo sapiens

<400>  42
ggagtttgca ttcttattca tcagg                                          25


<210>  43
<211>  25
<212>  DNA
<213>  Homo sapiens

<400>  43
ttgagcatgg atacagccag gccca                                          25


<210>  44
<211>  25
<212>  DNA
<213>  Homo sapiens

<400>  44
gaacctcccc aagatcagga atcct                                          25


<210>  45
<211>  25
<212>  DNA
<213>  Homo sapiens

<400>  45
tctacagcgg cattgtcact ttcat                                              25


<210>  46
<211>  25
<212>  DNA
<213>  Homo sapiens

<400>  46
gctgacaggt tggctggctc agcca                                              25


<210>  47
<211>  25
<212>  DNA
<213>  Homo sapiens

<400>  47
aaggcagtca atgctgccca ccaaa                                              25


<210>  48
<211>  25
<212>  DNA
<213>  Homo sapiens

<400>  48
aagaaaatac catgtcgacc agccg                                              25


<210>  49
<211>  25
<212>  DNA
<213>  Homo sapiens

<400>  49
gagcgaacgc tcgcacgagg tgtac                                              25


<210>  50
<211>  25
<212>  DNA
<213>  Homo sapiens

<400>  50
gtgggctcaa actgagcgcc tttgc                                              25


<210>  51
<211>  25
<212>  DNA
<213>  Homo sapiens

<400>  51
aaaactacgg ccatggcagc ctgga                                              25


<210>  52
<211>  25
<212>  DNA
<213>  Homo sapiens

<400>  52
aatacagctt atgcataacc tggga                                              25


<210>  53
<211>  25

<212>  DNA
<213>  Homo sapiens

<400>  53
agccctgttg atcactggac ataaa                                          25


<210>  54
<211>  25
<212>  DNA
<213>  Homo sapiens

<400>  54
tgaacgagtg tgcagacggt gccca                                          25


<210>  55
<211>  25
<212>  DNA
<213>  Homo sapiens

<400>  55
gtgattcaaa tgggttttcc acgct                                          25